# EUROPÄISCHE PATENTSCHRIFT

(11) **EP 0 760 967 B1**
(45) Veröffentlichungstag und Bekanntmachung des Hinweises auf die Patenterteilung: **13.05.1998**
(21) Anmeldenummer: 94931592.3
(22) Anmeldetag: 09.11.1994
(51) Int. Cl.: G02F 1/35, C09B 69/10, C09B 29/033

(54) **THIENOTHIENYLAZOANILINE IN DER NICHTLINEAREN OPTIK**
THIENOTHIENYL AZOANILINES IN NON-LINEAR OPTICS
THIENOTHIENYLAZOANILINES EN OPTIQUE NON LINEAIRE

(30) Priorität: 22.11.1993 DE 4339712
(43) Veröffentlichungstag der Anmeldung: 12.03.1997
(73) Patentinhaber: BASF AKTIENGESELLSCHAFT, 67056 Ludwigshafen (DE)
(72) Erfinder: BECKMANN, Stefan, D-68167 Mannheim (DE); ETZBACH, Karl-Heinz, D-67227 Frankenthal (DE); SENS, Ruediger, D-68165 Mannheim (DE)
(86) Internationale Anmeldenummer: EP9403685
(87) Internationale Veröffentlichungsnummer: WO9514957

(56) Entgegenhaltungen:
- EP-A- 0 251 114
- EP-A- 0 534 296
- EP-A- 0 535 490
- WO-A-91/09842

## Beschreibung

Die vorliegende Erfindung betrifft die Verwendung von Azofarbstoffen mit einer Diazokomponente, die sich von einem Aminothienothiophen ableitet, und einer Kupplungskomponente aus der Anilinreihe in der nichtlinearen Optik, Polymerisate, die sich von diesen Azofarbstoffen ableiten, deren Verwendung in der nichtlinearen Optik sowie neue Phenylthienothienylazoaniline.

Die nichtlinear optischen Eigenschaften organischer Verbindungen finden in vielen Bereichen der Optoelektronik Anwendung. Beispiele dafür sind Anwendungen in der Frequenzverdoppelung, in Phasenmodulatoren, optischen Verstärkern, Interferometern, optischen Schaltern oder in der Nachrichtentechnik.

Es ist allgemein bekannt, daß organische Materialien, insbesondere Polymere mit speziellen Chromophoren nichtlinear optische Eigenschaften aufweisen können, welche zum Teil größer sind als die vergleichbarer anorganischer Materialien. Die gegenwärtig am häufigsten angewandten Materialien sind anorganische Kristalle, z.B. aus Kaliumdihydrogenphosphat oder Lithiumniobat. Diese Kristalle sind aufwendig und mit hohen Kosten herzustellen sowie aufgrund ihrer starren Struktur nur schwierig in optischen Geräten anzuwenden. Ein weiterer Nachteil sind ihre geringen nichtlinearen Effekte.

Ein besonderer Vorteil geeigneter organischer Chromophore und ihrer Anwendung in polymeren Materialien liegt in ihrer einfachen Herstellung und Verarbeitung.

Die in der nichtlinearen Optik angewandten Chromophore werden in der Regel entweder in kristalliner oder polymergebundener Form eingesetzt.

Aus Angew. Chem., Band 96, Seiten 637 bis 651, 1984, ist die Anwendung von Stilbenderivaten oder speziellen Azofarbstoffen für diesen Zweck bekannt.

Aufgabe der vorliegenden Erfindung war es nun, geeignete Thienothienylazofarbstoffe bereitzustellen, die sich vorteilhaft für die Anwendung in polymeren nichtlinear optischen Systemen eignen. Insbesondere sollten solche Azoverbindungen große Hyperpolarisierbarkeitswerte, eine gute thermische Stabilität, gute Verträglichkeit mit den in nichtlinear optischen Systemen zur Anwendung kommenden Polymeren sowie gute Filmbildungseigenschaften mit Copolymeren aufweisen.

Es wurde nun gefunden, daß sich Azofarbstoffe der Formel I 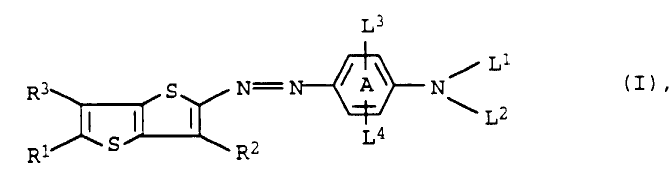 in der
der Ring A benzoanelliert sein kann,
- L¹ und L²: unabhängig voneinander jeweils Wasserstoff, C₁-C₁₀-Alkyl, das gegebenenfalls durch Phenyl, Hydroxy, Acryloyloxy oder Methacryloyloxy substituiert ist, oder Phenyl oder L¹ und L² zusammen mit dem sie verbindenden Stickstoffatom Pyrrolidinyl, Piperidinyl, Morpholinyl, Piperazinyl oder N- (C₁-C₄-Alkyl)piperazinyl,
- L³ und L⁴: unabhängig voneinander jeweils Wasserstoff, C₁-C₁₀-Alkyl, C₅-C₇-Cycloalkyl, C₁-C₁₀-Alkoxy, Cyano oder Halogen,
- R¹: Wasserstoff, Halogen, Nitro, Hydroxysulfonyl, Carbamoyl, Dicyanovinyl oder einen Rest der Formel CONX¹X², CH=N-X³, CH=C(NO₂)-X⁴ oder 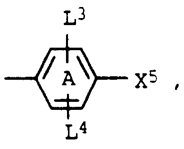 worin X¹ und X² unabhängig voneinander jeweils für C₁-C₁₀-Alkyl, das gegebenenfalls durch Phenyl substituiert ist, C₅-C₇-Cycloalkyl oder Phenyl, X³ für Hydroxy oder Phenylamino, X⁴ für Wasserstoff oder C₁-C₄-Alkyl und X⁵ für Wasserstoff, Nitro, Hydroxysulfonyl, Formyl, Cyano, C₁-C₄-Alkylsulfonyl, Dicyanovinyl oder einen Rest der Formel COX¹, COOX¹, CONX¹X², CH=N-X³ oder CH=C(NO₂)-X⁴, worin X¹, X², X³ und X⁴ jeweils die obengenannte Bedeutung besitzen, stehen und der Ring A und L³ und L⁴ jeweils die obengenannte Bedeutung besitzen,
- R²: Wasserstoff, Nitro, Cyano, Carboxyl oder einen Rest der Formel COX¹, COOX¹ oder CONX¹X², worin X¹ und X² jeweils die obengenannte Bedeutung besitzen, und
- R³: Wasserstoff, C₁-C₁₀-Alkyl, das gegebenenfalls durch Phenyl substituiert ist, C₅-C₇-Cycloalkyl, Phenyl oder C₁-C₁₀-Alkoxy bedeuten,
vorteilhaft zur Anwendung in der nichtlinearen Optik eignen.

Ähnliche Azofarbstoffe und deren Anwendung in der nichtlinearen Optik sind aus EP-A-0 535 490 bekannt.

Alle in der obengenannten Formel I auftretenden Alkylgruppen können sowohl geradkettig als auch verzweigt sein.

Wenn in der obengenannten Formel I substituierte Alkylgruppen auftreten, weisen diese in der Regel 1 oder 2 Substituenten auf.

Reste L¹, L², L³, L⁴, X¹, X², X⁴ und R³ sind z.B. Methyl, Ethyl, Propyl, Isopropyl, Butyl, Isobutyl oder sec-Butyl.

Reste L¹, L², L³, L⁴, X¹, X² und R³ sind weiterhin z.B. Pentyl, Isopentyl, Neopentyl, tert-Pentyl, Hexyl, 2-Methylpentyl, Heptyl, Octyl, 2-Ethylhexyl, Isooctyl, Nonyl, Isononyl, Decyl oder Isodecyl (die obigen Bezeichnungen Isooctyl, Isononyl und Isodecyl sind Trivialbezeichnungen und stammen von den nach der Oxosynthese erhaltenen Alkoholen - vgl. dazu Ullmanns Encyklopädie der technischen Chemie, 4. Auflage, Band 7, Seiten 215 bis 217, sowie Band 11, Seiten 435 und 436).

Reste L³, L⁴ und R³ sind weiterhin z.B. Cyclopentyl, Cyclohexyl, Cycloheptyl, Methoxy, Ethoxy, Propoxy, Isopropoxy, Butoxy, Isobutoxy, sec-Butoxy, Pentyloxy, Isopentyloxy, Neopentyloxy, tert-Pentyloxy, Hexyloxy, 2-Methylpentyloxy, Heptyloxy, Octyloxy, 2-Ethylhexyloxy, Isooctyloxy, Nonyloxy, Isononyloxy, Decyloxy oder Isodecyloxy.

Reste L¹, L² und R³ sind weiterhin z.B. Benzyl oder 1- oder 2-Phenylethyl.

Reste L¹ und L² sind weiterhin z.B. 2-Hydroxyethyl, 2- oder 3-Hydroxypropyl, 2- oder 4-Hydroxybutyl, 5-Hydroxypentyl, 6-Hydroxyhexyl, 7-Hydroxyheptyl, 8-Hydroxyoctyl, 9-Hydroxynonyl, 10-Hydroxydecyl, 2-Acryloyloxyethyl, 2-Methacryloyloxyethyl, 2- oder 3-Acryloyloxypropyl, 2- oder 3-Methacryloyloxypropyl, 2- oder 4-Acryloyloxybutyl, 2- oder 4-Methacryloyloxybutyl, 5-Acryloyloxypentyl, 5-Methacryloyloxypentyl, 6-Acryloyloxyhexyl, 6-Methacryloyloxyhexyl, 7-Acryloyloxyheptyl, 7-Methacryloyloxyheptyl, 8-Acryloyloxyoctyl, 8-Methacryloyloxyoctyl, 9-Acryloyloxynonyl, 9-Methacryloyloxynonyl, 10-Acryloyloxydecyl oder 10-Methacryloyloxydecyl.

Reste L³ und L⁴ sind weiterhin z.B. Fluor, Chlor, Brom oder Iod.

Reste X⁵ sind z.B. Methylsulfonyl, Ethylsulfonyl, Propylsulfonyl, Isopropylsulfonyl, Butylsulfonyl, Isobutylsulfonyl oder sec-Butylsulfonyl.

Bevorzugt ist die erfindungsgemäße Verwendung von Azofarbstoffen der Formel I, in der L¹ und L² unabhängig voneinander jeweils C₁-C₄-Alkyl, C₂-C₈-Hydroxyalkyl, C₂-C₈-Acryloyloxyalkyl oder C₂-C₈-Methacryloyloxyalkyl oder L¹ und L² zusammen mit dem sie verbindenden Stickstoffatom Pyrrolidinyl oder Piperidinyl bedeuten.

Weiterhin bevorzugt ist die erfindungsgemäße Verwendung von Azofarbstoffen der Formel I, in der L³ und L⁴ jeweils Wasserstoff bedeuten.

Weiterhin bevorzugt ist die erfindungsgemäße Verwendung von Azofarbstoffen der Formel I, in der der Ring A nicht benzoanelliert ist.

Weiterhin bevorzugt ist die erfindungsgemäße Verwendung von Azofarbstoffen der Formel I, in der R¹ Dicyanovinyl oder einen Rest der Formel 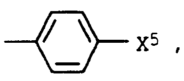 worin X⁵ die obengenannte Bedeutung besitzt, hierbei insbesondere Wasserstoff, Nitro, Formyl, Cyano, Methylsulfonyl oder Dicyanovinyl, R² Cyano und R³ Wasserstoff bedeuten.

Dicyanovinyl ist insbesondere 2,2-Dicyanovinyl.

Die Azofarbstoffe der Formel I sind teilweise bekannt und z.B. in der US-A-4 843 153 beschrieben.

Ein weiterer Gegenstand der vorliegenden Erfindung sind Azofarbstoffe der Formel Ia 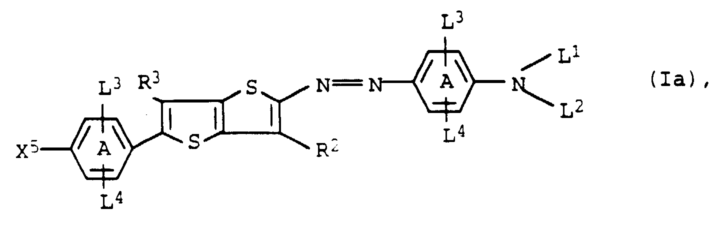 in der der Ring A benzoanelliert sein kann,
- L¹ und L²: unabhängig voneinander jeweils Wasserstoff, C₁-C₁₀-Alkyl, das gegebenenfalls durch Phenyl, Hydroxy, Acryloyloxy oder Methacryloyloxy substituiert ist, oder Phenyl oder L¹ und L² zusammen mit dem sie verbindenden Stickstoffatom Pyrrolidinyl, Piperidinyl, Morpholinyl, Piperazinyl oder N-(C₁-C₄-Alkyl)piperazinyl,
- L³ und L⁴: unabhängig voneinander jeweils Wasserstoff, C₁-C₁₀-Alkyl, C₅-C₇-Cycloalkyl, C₁-C₁₀-Alkoxy, Cyano oder Halogen,
- X⁵: Wasserstoff, Nitro, Hydroxysulfonyl, Formyl, Cyano oder C₁-C₄-Alkylsulfonyl, Dicyanovinyl oder einen Rest der Formel COX¹, COOX¹, CONX¹X², CH=N-X³ oder CH=C(NO₂)-X⁴, worin X¹ und X² unabhängig voneinander jeweils für C₁-C₁₀-Alkyl, das gegebenenfalls durch Phenyl substituiert ist, C₅-C₇-Cycloalkyl oder Phenyl, X³ für Hydroxy oder Phenylamino und X⁴ für Wasserstoff oder C₁-C₄-Alkyl stehen,
- R²: Wasserstoff, Nitro, Cyano, Carboxyl oder einen Rest der Formel COX¹, COOX¹ oder CONX¹X², worin X¹ und X² jeweils die obengenannte Bedeutung besitzen, und
- R³: Wasserstoff, C₁-C₁₀-Alkyl, das gegebenenfalls durch Phenyl substituiert ist, C₅-C₇-Cycloalkyl, Phenyl oder C₁-C₁₀-Alkoxy bedeuten.

Bevorzugt sind Azofarbstoffe der Formel Ia, in der L¹ und L² unabhängig voneinander jeweils C₁-C₄-Alkyl, C₂-C₈-Hydroxyalkyl, C₂-C₈-Acryloyloxyalkyl oder C₂-C₈-Methacryloyloxyalkyl oder L¹ und L² zusammen mit dem sie verbindenden Stickstoffatom Pyrrolidinyl oder Piperidinyl bedeuten.

Weiterhin bevorzugt sind Azofarbstoffe der Formel Ia, in der L³ und L⁴ jeweils Wasserstoff bedeuten.

Weiterhin bevorzugt sind Azofarbstoffe der Formel Ia, in der der Ring A nicht benzoanelliert ist.

Weiterhin bevorzugt sind Azofarbstoffe der Formel Ia, in der X⁵ Wasserstoff, Nitro, Formyl, Cyano, Methylsulfonyl oder Dicyanovinyl, hierbei insbesondere Cyano, R² Cyano und R³ Wasserstoff bedeuten.

Die Herstellung der Azofarbstoffe der Formel I kann nach an sich bekannten Methoden erfolgen. Beispielsweise kann man ein Amin der Formel II 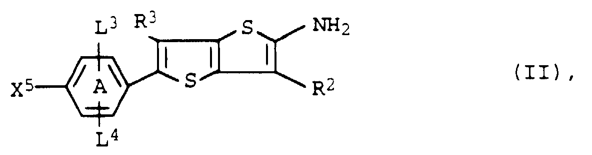 in der der Ring A, L³, L⁴, X⁵, R² und R³ jeweils die obengenannte Bedeutung besitzen, diazotieren und mit einer Kupplungskomponente der Formel III 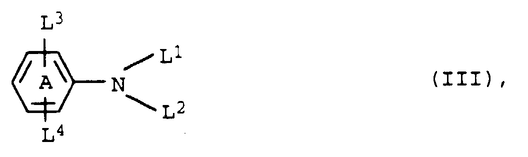 in der der Ring A, L¹, L², L³ und L⁴ jeweils die obengenannte Bedeutung besitzen, kuppeln.

Die vorliegende Erfindung betrifft weiterhin Azofarbstoffe enthaltende Polymerisate, die als charakteristische Monomereinheiten einen bivalenten Rest, der sich von einem Azofarbstoff der Formel I ableitet, sowie Reste der Formeln IV, V und VI 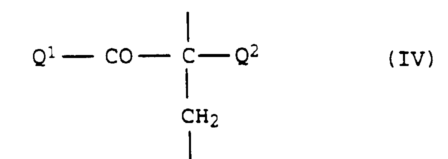 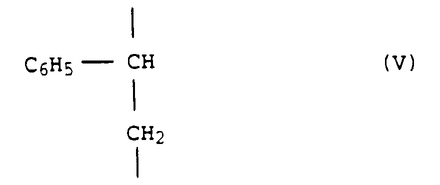 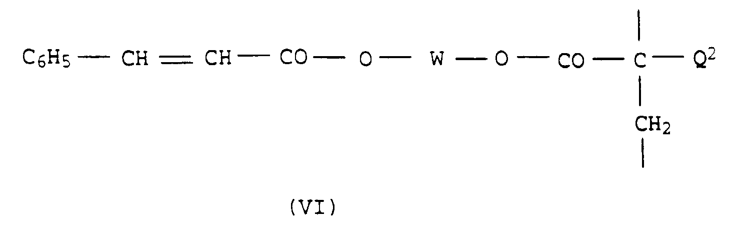 aufweisen, worin
- Q¹: Hydroxy, C₁-C₆-Alkoxy, Oxiranylmethoxy, Phenoxy, Amino oder C₁-C₄-Mono- oder Dialkylamino,
- Q²: Wasserstoff oder Methyl und
- W: C₂-C₁₀-Alkylen bedeuten,
wobei der Anteil der Monomereinheiten der bivalenten Reste, die sich von Formel I ableiten, 1 bis 100 Mol-%, der der Formel IV 0 bis 99 Mol-%, der der Formel V 0 bis 99 Mol-% und der der Formel VI 0 bis 75 Mol-%, jeweils bezogen auf das Polymerisat, und das mittlere Molekulargewicht des Polymerisats 1.000 bis 500.000 betragen.

Vorzugsweise gehorcht ein bivalenter Rest, der sich von einem Azofarbstoff der Formel I ableitet, der Formel VII 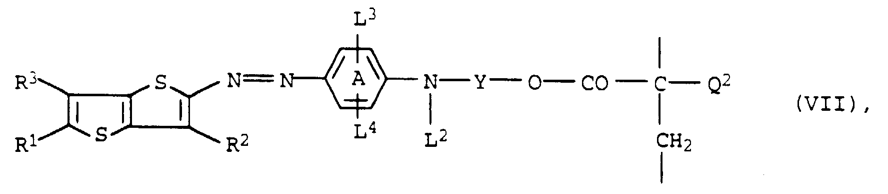 worin Y C₂-C₁₀-Alkylen bedeutet und der Ring A, L², L³, L⁴, R¹, R² und Q² jeweils die obengenannte Bedeutung besitzen.

Die Herstellung der neuen Polymerisate kann nach an sich bekannten Methoden, wie sie z.B. in J. Polymer Sci., Part A, Polymer Chem., Band 28, Seiten 1 bis 13, 1990, beschrieben sind, erfolgen.

Zweckmäßig setzt man dabei einen entsprechenden Azofarbstoff der Formel I mit einer Acrylverbindung der Formel VIII 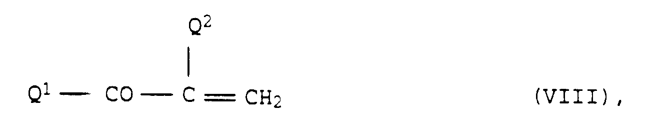 in der Q¹ und Q² jeweils die obengenannte Bedeutung besitzen, Styrol und einem Zimtsäureester der Formel IX 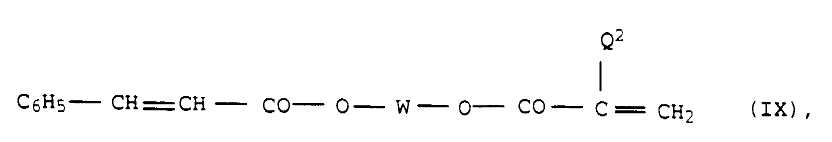 in der Q² und W jeweils die obengenannte Bedeutung besitzen, im obengenannten Molverhältnis in einem inerten Lösungsmittel (z.B. Toluol oder Xylol) in Gegenwart eines Radikalstarters (z.B. Azobisisobutyronitril) um.

Auch die Azofarbstoffe der Formel I enthaltenden Polymerisate eignen sich vorteilhaft zur Anwendung in der nichtlinearen Optik.

Die erfindungsgemäßen Verbindungen sind thermisch stabil und verfügen über besonders große molekulare Hyperpolarisierbarkeitswerte (β). Außerdem weisen die Farbstoffe eine gute Verträglichkeit mit den in nichtlinear optischen Systemen zur Anwendung kommenden Polymeren sowie gute Filmbildungseigenschaften in Copolymeren auf.

Die Bestimmung der molekularen Hyperpolarisierbarkeit kann z.B. nach der Solvatochromiemeßmethode (siehe beispielsweise Z. Naturforschung, Band 20a, Seite 1441 bis 1471, 1965, oder J. Org. Chem., Band 54, Seite 3775 bis 3778, 1989, erfolgen. Man bestimmt dabei die Lage der Absorptionsbande einer Verbindung in verschiedenen Lösungsmitteln, z.B. in Dioxan oder Dimethylsulfoxid. Die Verschiebung der Absorptionsbande ist dann direkt proportional dem β-Wert, d.h. Verbindungen mit großer solvatochromer Verschiebung weisen eine große molekulare Hyperpolyrisierbarkeit auf und eignen sich daher gut für die Anwendung in nichtlinear optischen Systemen (siehe beispielsweise Chemistry and Industry, Seiten 600 bis 608, 1990).

Insbesondere ist hierbei die Eignung der neuen Stoffe in der Nachrichtentechnik, in elektrooptischen Modulatoren (z.B. Machzehnder-Inferometer), in optischen Schaltern, bei der Frequenzmischung oder in Wellenleitern hervorzuheben.

Die folgenden Beispiele sollen die Erfindung näher erläutern.

### Beispiel 1

1,0 g (0,004 mol) der Verbindung der Formel 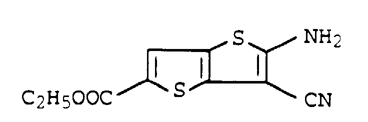 (hergestellt gemäß Beispiel 3 der US-A-4 843 153) wurden bei 0 bis 5°C in 20 ml Eisessig/Propionsäure (17:3 v/v) suspendiert und mit 1,6 g Nitrosylschwefelsäure versetzt. Man rührte 1 h bei 5°C und gab die entstehende Lösung zu einem Gemisch von 0,65 g (0,004 mol) N,N-Diethylanilin in 5 ml Eisessig/Propionsäure (17:3 v/v). Man rührte dann 30 min und gab auf 250 ml Eiswasser. Man saugte ab und wusch mit Wasser. Nach dem Trocknen unter vermindertem Druck bei 50°C erhielt man 1,2 g eines roten Farbstoffs der Formel

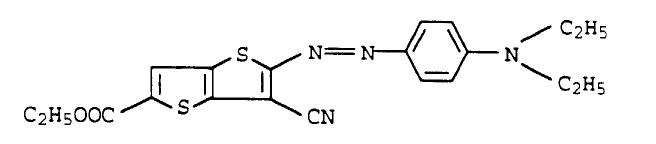

### Beispiel 2

1,0 g (0,004 mol) der Verbindung der Formel 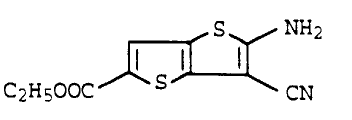 wurden bei 0 bis 5°C in 20 ml Eisessig/Propionsäure (17:3 v/v) suspendiert und mit 1,6 g Nitrosylschwefelsäure versetzt. Man rührte 1 h bei 5°C und gab die entstehende Lösung zu einem Gemisch von 0,65 g (0,004 mol) N-Phenylpyrrolidin und 5 ml Eisessig/Propionsäure (17:3 v/v). Man rührte 30 min bei 0 bis 5°C und gab auf 250 ml Eiswasser. Man saugte ab und wusch mit 1 1 Wasser nach. Nach dem Trocknen unter vermindertem Druck bei 50°C erhielt man 1,13 g eines roten Farbstoffs der Formel

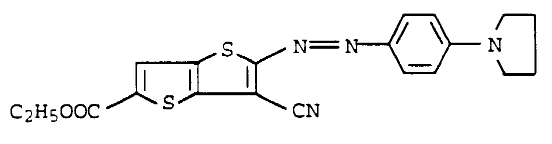

### Beispiel 3

1,0 g (0,004 mol) der Verbindung der Formel 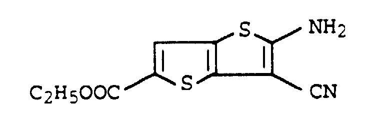 wurden bei 0 bis 5°C in 20 ml Eisessig/Propionsäure (17:3 v/v) suspendiert und mit 1,16 g Nitrosylschwefelsäure versetzt. Man rührte 1 h bei 5°C und gab die entstehende Lösung zu einem Gemisch von 1,088 g (0,004 mol) der Verbindung der Formel 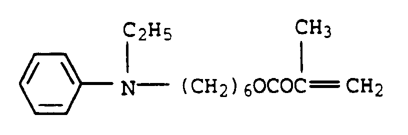 und 5 ml Eisessig/Propionsäure (17:3 v/v). Man rührte 30 min bei 0 bis 5°C und gab auf 250 ml Eiswasser. Man saugte ab und wusch mit 1 1 Wasser nach. Nach dem Trocknen unter vermindertem Druck erhielt man 1,92 g eines Farbstoffs der Formel

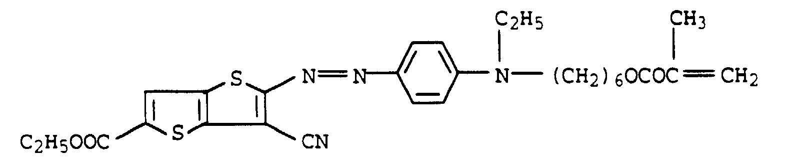

### Beispiel 4

a) 73,15 g (0,21 mol) der Verbindung der Formel 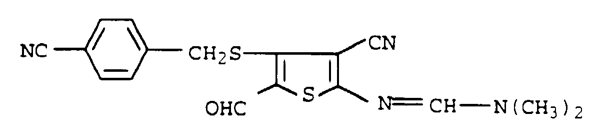 wurden in 370 ml N,N-Dimethylformamid mit 25,5 g (0,33 mol) Natriumacetat 7 h auf 100°C erhitzt. Anschließend wurde der ausgefallene Niederschlag abgesaugt, mit Wasser gewaschen und bei 50°C unter vermindertem Druck getrocknet. Man erhielt 24,6 g der Verbindung der Formel 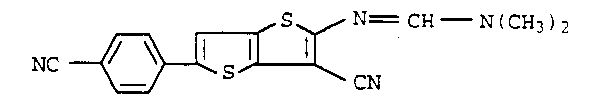 Fp.: 243 bis 244°C

| Analyse: | | | | |
|---|---|---|---|---|
| ber | C 67,08 | H 3,97 | N 18,41 | S 10,53 |
| gef | C 67,14 | H 4,04 | N 17,88 | S 10,38 |

b) 7,8 g (0,023 mol) der in Beispiel 4a beschriebenen Verbindung wurden in 40 ml N-Methylpyrrolidon unter Zusatz von 4,6 g konz. Salzsäure 2 h auf 100°C erhitzt. Anschließend wurde der Reaktionsansatz auf 500 g Eis/Wasser gefällt, abgesaugt, mit 200 ml Wasser gewaschen und bei 50°C unter vermindertem Druck getrocknet. Man erhielt 6,6 g einer Verbindung der Formel 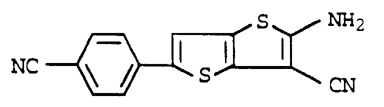
c) 4,78 g (0,017 mol) der in Beispiel 4b beschriebenen Verbindung wurden bei 0 bis 5°C in 170 ml Eisessig/Propionsäure (17:3 v/v) mit 5,34 g Nitrosylschwefelsäure versetzt. Man rührte anschließend 2 h bei 0 bis 5°C und gab in der Kälte 2,94 g (0,0187) N-Ethyl-N-(2-hydroxyethyl)anilin zu. Nach 2 h wurde abgesaugt, mit Wasser gewaschen und unter vermindertem Druck getrocknet. Man erhielt 5,0 g eines Farbstoffs der Formel 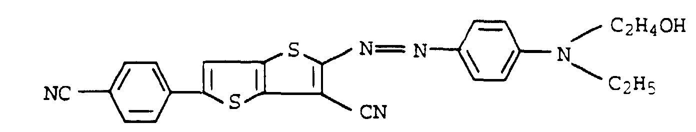 Fp.: 272 bis 273°C

| Analyse: | | | | | |
|---|---|---|---|---|---|
| ber | C 63,00 | H 4,19 | O 3,5 | N 15,31 | S 14,01 |
| gef | C 63,20 | H 4,20 | O 3,8 | N 14,90 | S 13,90 |

In analoger Weise werden die folgenden Verbindungen erhalten.

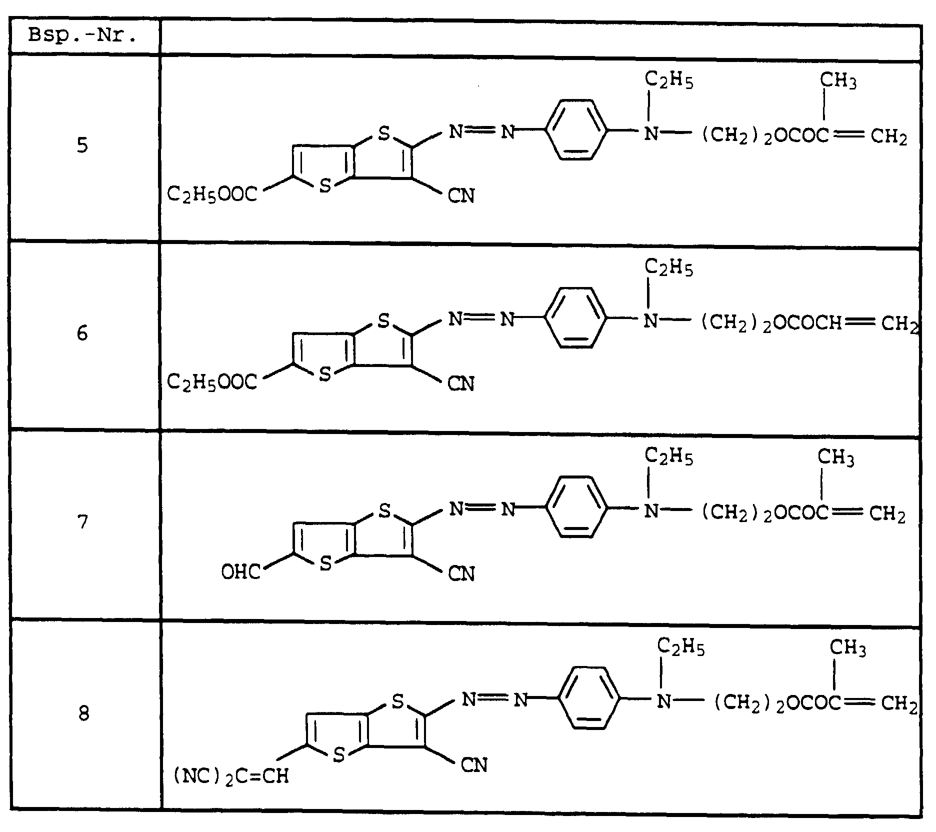

Nach der in Z. Naturforschung, Band 20a, Seiten 1441 bis 1471, 1965, beschriebenen Methode wurde das Absorptionsmaximum der einzelnen Farbstoffe jeweils in Dioxan und Dimethylsulfoxid (DMSO) gemessen und dann die solvatochrome Verschiebung 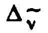 [cm⁻¹] bestimmt.

Die jeweiligen Meßergebnisse sind in der folgenden Tabelle aufgeführt.

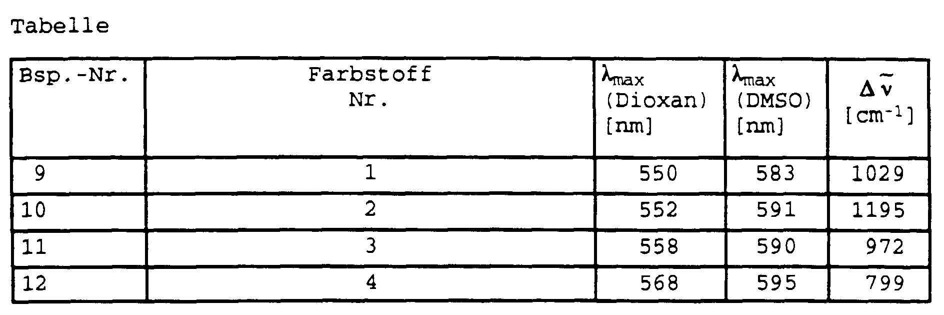

## Patentansprüche

1. Verwendung von Azofarbstoffen der Formel I 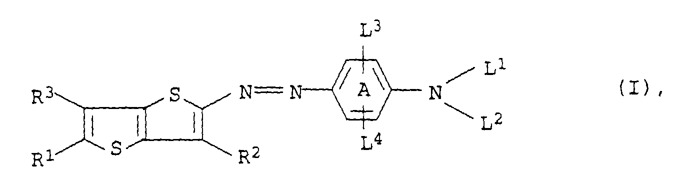 in der
der Ring A benzoanelliert sein kann,
L¹ und L² unabhängig voneinander jeweils Wasserstoff, C₁-C₁₀-Alkyl, das gegebenenfalls durch Phenyl, Hydroxy, Acryloyloxy oder Methacryloyloxy substituiert ist, oder Phenyl oder L¹ und L² zusammen mit dem sie verbindenden Stickstoffatom Pyrrolidinyl, Piperidinyl, Morpholinyl, Piperazinyl oder N-(C₁-C₄-Alkyl)piperazinyl,
L³ und L⁴ unabhängig voneinander jeweils Wasserstoff, C₁-C₁₀-Alkyl, C₅-C₇-Cycloalkyl, C₁-C₁₀-Alkoxy, Cyano oder Halogen,
R¹ Wasserstoff, Nitro, Hydroxysulfonyl, Carbamoyl, Dicyanovinyl oder einen Rest der Formel CONX¹X², CH=N-X³, CH=C(NO₂)-X⁴ oder 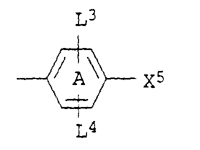 worin X¹ und X² unabhängig voneinander jeweils für C₁-C₁₀-Alkyl, das gegebenenfalls durch Phenyl substituiert ist, C₅-C₇-Cycloalkyl oder Phenyl, X³ für Hydroxy oder Phenylamino, X⁴ für Wasserstoff oder C₁-C₄-Alkyl und X⁵ für Wasserstoff, Nitro, Hydroxysulfonyl, Formyl, Cyano, C₁-C₄-Alkylsulfonyl, Dicyanovinyl oder einen Rest der Formel COX¹, COOX¹, CONX¹X², CH=N-X³ oder CH=C(NO₂)-X⁴, worin X¹, X², X³ und X⁴ jeweils die obengenannte Bedeutung besitzen, stehen und der Ring A und L³ und L⁴ jeweils die obengenannte Bedeutung besitzen,
R² Wasserstoff, Nitro, Cyano, Carboxyl oder einen Rest der Formel COX¹, COOX¹ oder CONX¹X², worin X¹ und X² jeweils die obengenannte Bedeutung besitzen, und
R³ Wasserstoff, C₁-C₁₀-Alkyl, das gegebenenfalls durch Phenyl substituiert ist, C₅-C₇-Cycloalkyl, Phenyl oder C₁-C₁₀-Alkoxy bedeuten,
in der nichtlinearen Optik.

2. Verwendung von Azofarbstoffen nach Anspruch 1, wobei L¹ und L² unabhängig voneinander jeweils C₁-C₄-Alkyl, C₂-C₈-Hydroxyalkyl, C₂-C₈-Acryloyloxyalkyl oder C₂-C₈-Methacryloyloxyalkyl oder L¹ und L² zusammen mit dem sie verbindenden Stickstoffatom Pyrrolidinyl oder Piperidinyl bedeuten.

3. Verwendung von Azofarbstoffen nach Anspruch 1, wobei L³ und L⁴ jeweils Wasserstoff bedeuten.

4. Verwendung von Azofarbstoffen nach Anspruch 1, wobei R¹ Dicyanovinyl oder einen Rest der Formel 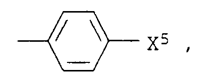 worin X⁵ die in Anspruch 1 genannte Bedeutung besitzt, R² Cyano und R³ Wasserstoff bedeuten.

5. Azofarbstoffe der Formel Ia 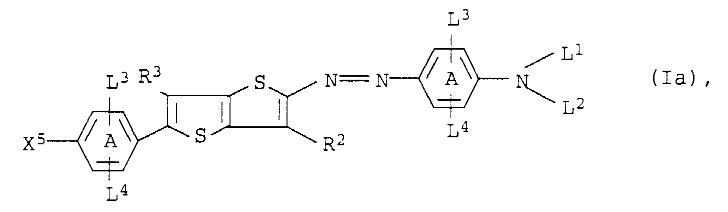 in der
der Ring A benzoanelliert sein kann,
L¹ und L² unabhängig voneinander jeweils Wasserstoff, C₁-C₁₀-Alkyl, das gegebenenfalls durch Phenyl, Hydroxy, Acryloyloxy oder Methacryloyloxy substituiert ist, oder Phenyl oder L¹ und L² zusammen mit dem sie verbindenden Stickstoffatom Pyrrolidinyl, Piperidinyl, Morpholinyl, Piperazinyl oder N-(C₁-C₄-Alkyl)piperazinyl,
L³ und L⁴ unabhängig voneinander jeweils Wasserstoff, C₁-C₁₀-Alkyl, C₅-C₇-Cycloalkyl, C₁-C₁₀-Alkoxy, Cyano oder Halogen,
X⁵ Wasserstoff, Nitro, Hydroxysulfonyl, Formyl, Cyano, C₁-C₄-Alkylsulfonyl, Dicyanovinyl oder einen Rest der Formel COX¹, COOX¹, CONX¹X², CH=N-X³ oder CH=C(NO₂)-X⁴, worin X¹ und X² unabhängig voneinander jeweils für C₁-C₁₀-Alkyl, das gegebenenfalls durch Phenyl substituiert ist, C₅-C₇-Cycloalkyl oder Phenyl, X³ für Hydroxy oder Phenylamino und X⁴ für Wasserstoff oder C₁-C₄-Alkyl stehen,
R² Wasserstoff, Nitro, Cyano, Carboxyl oder einen Rest der Formel COX¹, COOX¹ oder CONX¹X², worin X¹ und X² jeweils die obengenannte Bedeutung besitzen, und
R³ Wasserstoff, C₁-C₁₀-Alkyl, das gegebenenfalls durch Phenyl substituiert ist, C₅-C₄-Cycloalkyl, Phenyl oder C₁-C₁₀-Alkoxy bedeuten.

6. Azofarbstoffe nach Anspruch 5, wobei L¹ und L² unabhängig voneinander jeweils C₁-C₄-Alkyl, C₂-C₈-Hydroxyalkyl, C₂-C₈-Acryloyloxyalkyl oder C₂-C₈-Methacryloyloxyalkyl oder L¹ und L² zusammen mit dem sie verbindenden Stickstoffatom Pyrrolidinyl oder Piperidinyl bedeuten.

7. Azofarbstoffe nach Anspruch 5, wobei L³ und L⁴ jeweils Wasserstoff bedeuten.

8. Azofarbstoffe nach Anspruch 5, wobei X⁵ Wasserstoff, Nitro, Formyl, Cyano, Methylsulfonyl oder Dicyanovinyl, R² Cyano und R³ Wasserstoff bedeuten.

9. Azofarbstoffe enthaltende Polymerisate, die als charakteristische Monomereinheiten einen bivalenten Rest, der sich von einem Azofarbstoff der Formel I gemäß Anspruch 1 ableitet, sowie Reste der Formeln IV, V und VI 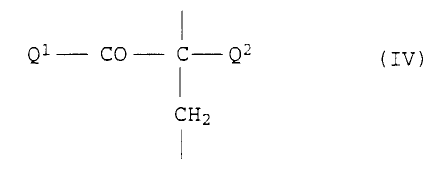 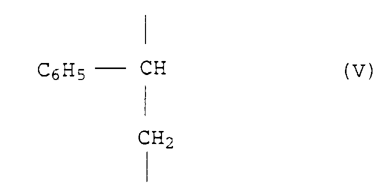 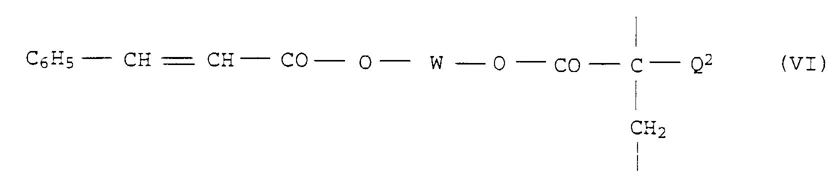 aufweisen, worin
Q¹ Hydroxy, C₁-C₆-Alkoxy, Oxiranylmethoxy, Phenoxy, Amino oder C₁-C₄-Mono- oder Dialkylamino,
Q² Wasserstoff oder Methyl und
W C₂-C₁₀-Alkylen bedeuten,
wobei der Anteil der Monomereinheiten der bivalenten Reste, die sich von Formel I ableiten, 1 bis 100 Mol-%, der der Formel IV 0 bis 99 Mol-%, der der Formel V 0 bis 99 Mol-% und der der Formel VI 0 bis 75 Mol-%, jeweils bezogen auf das Polymerisat, und das mittlere Molekulargewicht des Polymerisats 1.000 bis 500.000 betragen.

10. Verwendung der Azofarbstoffe enthaltenden Polymerisate gemäß Anspruch 9 in der nichtlinearen Optik.

## Claims

1. The use in nonlinear optics of azo dyes of the formula I 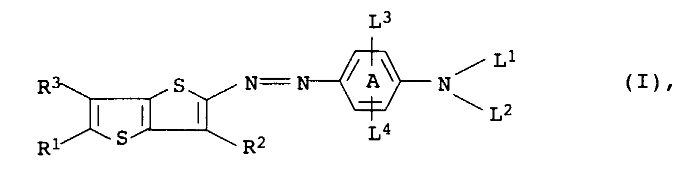 where
the ring A may be benzofused,
L¹ and L² are each independently of the other hydrogen, unsubstituted or phenyl-, hydroxyl-, acryloyloxy- or methacryloyloxy-substituted C₁-C₁₀-alkyl, or phenyl, or L¹ and L² are together with the nitrogen atom joining them together pyrrolidinyl, piperidinyl, morpholinyl, piperazinyl or N-(C₁-C₄-alkyl)piperazinyl,
L³ and L⁴ are each independently of the other hydrogen, C₁-C₁₀-alkyl, C₅-C₇-cycloalkyl, C₁-C₁₀-alkoxy, cyano or halogen,
R¹ is hydrogen, nitro, hydroxysulfonyl, carbamoyl, dicyanovinyl or a radical of the formula CONX¹X², CH=N-X³, CH=C(NO₂)-X⁴ or 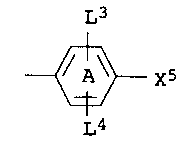 where X¹ and X² are each independently of the other C₁-C₁₀-alkyl, which may be phenyl-substituted, C₅-C₇-cycloalkyl or phenyl, X³ is hydroxyl or phenylamino, X⁴ is hydrogen or C₁-C₄-alkyl, and X⁵ is hydrogen, nitro, hydroxysulfonyl, formyl, cyano, C₁-C₄-alkylsulfonyl, dicyanovinyl or a radical of the formula COX¹, COOX¹, CONX¹X², CH=N-X³ or CH=C(NO₂)-X⁴, where X¹, X², X³ and X⁴ are each as defined above, and the ring A and L³ and L⁴ are each as defined above,
R² is hydrogen, nitro, cyano, carboxyl or a radical of the formula COX¹, COOX¹ or CONX¹X², where X¹ and X² are each as defined above, and
R³ is hydrogen, C₁-C₁₀-alkyl, which may be phenyl-substituted, C₅-C₇-Cycloalkyl, phenyl or C₁-C₁₀-alkoxy.

2. A use as claimed in claim 1, wherefor L¹ and L² are each independently of the other C₁-C₄-alkyl, C₂-C₈-hydroxyalkyl, C₂-C₈-acryloyloxyalkyl or C₂-C₈-methacryloyloxyalkyl, or L¹ and L² are together with the nitrogen atom joining them together pyrrolidinyl or piperidinyl.

3. A use as claimed in claim 1, wherefor L³ and L⁴ are each hydrogen.

4. A use as claimed in claim 1, wherefor R¹ is dicyanovinyl or a radical of the formula 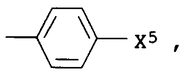 wherein X⁵ is as defined in claim 1, R² is cyano and R³ is hydrogen.

5. Azo dyes of the formula Ia 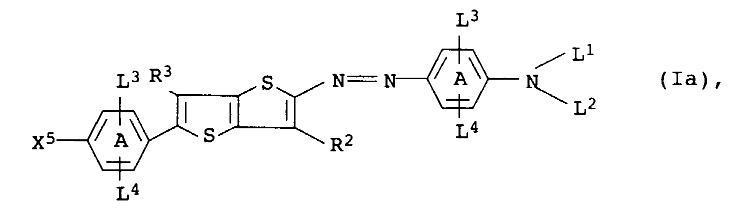 where
the ring A may be benzofused,
L¹ and L² are each independently of the other hydrogen, unsubstituted or phenyl-, hydroxyl-, acryloyloxy- or methacryloyloxy-substituted C₁-C₁₀-alkyl, or phenyl, or L¹ and L² are together with the nitrogen atom joining them together pyrrolidinyl, piperidinyl, morpholinyl, piperazinyl or N-(C₁-C₄-alkyl)piperazinyl,
L³ and L⁴ are each independently of the other hydrogen, C₁-C₁₀-alkyl, C₅-C₇-cycloalkyl, C₁-C₁₀-alkoxy, cyano or halogen,
X⁵ is hydrogen, nitro, hydroxysulfonyl, formyl, cyano, C₁-C₄-alkylsulfonyl, dicyanovinyl or a radical of the formula COX¹, COOX¹, CONX¹X², CH=N-X³ or CH=C(NO₂)-X⁴, where X¹ and X² are each independently of the other C₁-C₁₀-alkyl, which may be phenyl-substituted, C₅-C₇-cycloalkyl or phenyl, X³ is hydroxyl or phenylamino and X⁴ is hydrogen or C₁-C₄-alkyl,
R² is hydrogen, nitro, cyano, carboxyl or a radical of the formula COX¹, COOX¹ or CONX¹X², where X¹ and X² are each as defined above, and
R³ is hydrogen, C₁-C₁₀-alkyl, which may be phenyl-substituted, C₅-C₇-cycloalkyl, phenyl or C₁-C₁₀-alkoxy.

6. Azo dyes as claimed in claim 5, wherein L¹ and L² are each independently of the other C₁-C₄-alkyl, C₂-C₈-hydroxyalkyl, C₂-C₈-acryloyloxyalkyl or C₂-C₈-methacryloyloxyalkyl, or L¹ and L² are together with the nitrogen atom joining them together pyrrolidinyl or piperidinyl.

7. Azo dyes as claimed in claim 5, wherein L³ and L⁴ are each hydrogen.

8. Azo dyes as claimed in claim 5, wherein X⁵ is hydrogen, nitro, formyl, cyano, methylsulfonyl or dicyanovinyl, R² is cyano and R³ is hydrogen.

9. Azo dye polymers containing as characteristic monomer units a bivalent radical derived from an azo dye of the formula I as claimed in claim 1 and also radicals of the formulae IV, V and VI 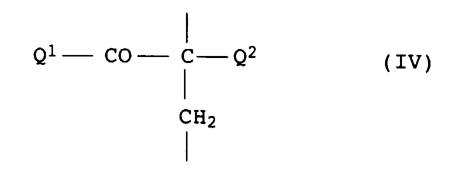 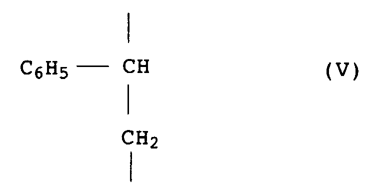 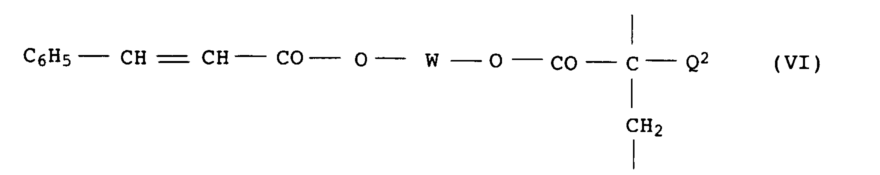 where
Q¹ is hydroxyl, C₁-C₆-alkoxy, oxiranylmethoxy, phenoxy, amino or mono- or di(C₁-C₄-alkyl)amino,
Q² is hydrogen or methyl, and
W is C₂-C₁₀-alkylene,
wherein the proportion of the monomer units of the bivalent radicals derived from the formula I is from 1 to 100 mol%, that of those of the formula IV from 0 to 99 mol%, that of those of the formula V from 0 to 99 mol% and that of those of the formula VI from 0 to 75 mol%, in each case based on the polymer, the average molecular weight of the polymer being from 1000 to 500,000.

10. The use of the azo dye polymers of claim 9 in nonlinear optics.

## Revendications

1. Utilisation de colorants azoïques de formule I 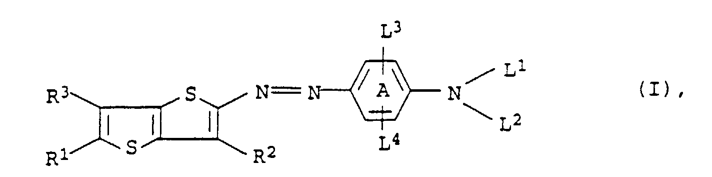 dans laquelle
le cycle A peut être benzocondensé,
L¹ et L² représentent à chaque fois indépendamment l'un de l'autre un atome d'hydrogène, un groupement alkyle en C₁-C₁₀ éventuellement substitué par un groupement phényle, hydroxy, acryloyloxy ou méthacryloyloxy, ou un groupement phényle, ou bien L¹ et L² représentent ensemble, avec l'atome d'azote qui les relie, un groupement pyrrolidinyle, pipéridinyle, morpholinyle, pipérazinyle ou N-(alkyle en C₁-C₄)pipérazinyle,
L³ et L⁴ représentent à chaque fois indépendamment l'un de l'autre un atome d'hydrogène, un groupement alkyle en C₁-C₁₀, un groupement cycloalkyle en C₅-C₇, un groupement alcoxy en C₁-C₁₀, un groupement cyano ou un atome d'halogène,
R¹ représente un atome d'hydrogène, un groupement nitro, un groupement hydroxysulfonyle, un groupement carbamoyle, un groupement dicyanovinyle ou un reste de formule CONX¹X², CH = N-X³, CH = C(NO₂)-X⁴ ou 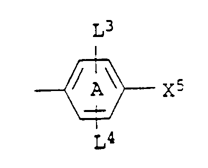 où X¹ et X² représentent chacun indépendamment l'un de l'autre un groupement alkyle en C₁-C₁₀, éventuellement substitué par un groupement phényle, un groupement cycloalkyle en C₅-C₇ ou phényle, X³ représente un groupement hydroxy ou phénylamino, X⁴ représente un atome d'hydrogène ou un groupement alkyle en C₁-C₄ et X⁵ représente un atome d'hydrogène, un groupement nitro, hydroxysulfonyle, formyle, cyano, (alkyle en C₁-C₄)sulfonyle, dicyanovinyle ou un reste de formule COX¹, COOX¹, CONX¹X², CH = N-X³ ou CH = C(NO₂)-X⁴, où X¹, X², X³ et X⁴ prennent à chaque fois la signification susmentionnée, le cycle A et L³ et L⁴ prennant chacun la signification susmentionnée,
R² représente un atome d'hydrogène, un groupement nitro, cyano, carboxyle ou un reste de formule COX¹, COOX¹ ou CONX¹X², où X¹ et X² prennent chacun la signification susmentionnée, et
R³ représente un atome d'hydrogène, un groupement alkyle en C₁-C₁₀ éventuellement substitué par un groupement phényle, un groupement cycloalkyle en C₅-C₇, un groupement phényle ou alcoxy en C₁-C₁₀,
en optique non linéaire.

2. Utilisation de colorants azoïques selon la revendication 1, où L¹ et L² représentent chacun indépendamment l'un de l'autre un groupement alkyle en C₁-C₄, hydroxyalkyle en C₂-C₈, acryloyloxyalkyle en C₂-C₈ ou méthacryloyloxyalkyle en C₂-C₈, ou bien L¹ et L² représentent ensemble, avec l'atome d'azote qui les relie, un groupement pyrrolidinyle ou pipéridinyle.

3. Utilisation de colorants azoïques selon la revendication 1, où L³ et L⁴ représentent chacun un atome d'hydrogène.

4. Utilisation de colorants azoïques selon la revendication 1, où R¹ représente un groupement dicyanovinyle ou un reste de formule 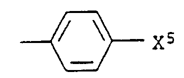 dans laquelle X⁵ prend la signification mentionnée dans la revendication 1, R² représente un groupement cyano et R³ un atome d'hydrogène.

5. Colorants azoïques de formule Ia 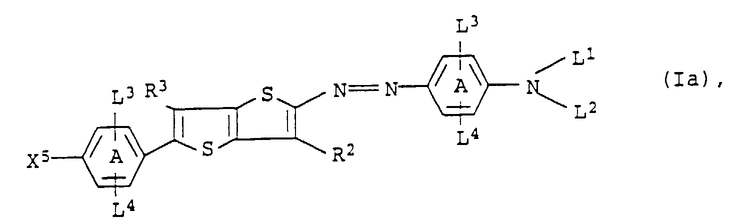 dans laquelle
le cycle A peut être benzocondensé,
L¹ et L² représentent à chaque fois indépendamment l'un de l'autre un atome d'hydrogène, un groupement alkyle en C₁-C₁₀ éventuellement substitué par un groupement phényle, hydroxy, acryloyloxy ou méthacryloyloxy, ou un groupement phényle, ou bien L¹ et L² représentent ensemble, avec l'atome d'azote qui les relie, un groupement pyrrolidinyle, pipéridinyle, morpholinyle, pipérazinyle ou N-(alkyle en C₁-C₄)pipérazinyle,
L³ et L⁴ représentent à chaque fois indépendamment l'un de l'autre un atome d'hydrogène, un groupement alkyle en C₁-C₁₀, un groupement cycloalkyle en C₅-C₇, un groupement alcoxy en C₁-C₁₀, un groupement cyano ou un atome d'halogène,
X⁵ représente un atome d'hydrogène, un groupement nitro, hydroxysulfonyle, formyle, cyano, (alkyle en C₁-C₄)sulfonyle, dicyanovinyle ou un reste de formule COX¹, COOX¹, CONX¹X², CH = N-X³ ou CH = C(NO₂)-X⁴ où X¹ et X² représentent chacun indépendamment l'un de l'autre un groupement alkyle en C₁-C₁₀, éventuellement substitué par un groupement phényle, un groupement cycloalkyle en C₅-C₇ ou phényle, X³ représente un groupement hydroxy ou phénylamino et X⁴ représente un atome d'hydrogène ou un groupement alkyle en C₁-C₄,
R² représente un atome d'hydrogène, un groupement nitro, cyano. carboxyle ou un reste de formule COX¹, COOX¹ ou CONX¹X², où X¹ et X² prennent chacun la signification susmentionnée, et
R³ représente un atome d'hydrogène, un groupement alkyle en C₁-C₁₀ éventuellement substitué par un groupement phényle, un groupement cycloalkyle en C₅-C₇, un groupement phényle ou alcoxy en C₁-C₁₀,

6. Colorants azoïques selon la revendication 5, où L¹ et L² représentent chacun indépendamment l'un de l'autre un groupement alkyle en C₁-C₄, hydroxyalkyle en C₂-C₈, acryloyloxyalkyle en C₂-C₈ ou méthacryloyloxyalkyle en C₂-C₈ ou bien L¹ et L² représentent ensemble, avec l'atome d'azote qui les relie, un groupement pyrrolidinyle ou pipéridinyle.

7. Colorants azoïques selon la revendication 5, où L³ et L⁴ représentent chacun un atome d'hydrogène.

8. Colorants azoïques selon la revendication 5, où X⁵ représente un atome d'hydrogène, un groupement nitro, formyle, cyano, méthylsulfonyle ou dicyanovinyle, R² représente un groupement cyano et R³ un atome d'hydrogène.

9. Polymères contenant des colorants azoïques, présentant en tant que motifs monomères caractéristiques un reste bivalent dérivant d'un colorant azoïque de formule I selon la revendication 1, ainsi que les restes de formules IV, V, VI 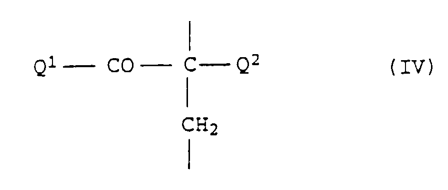 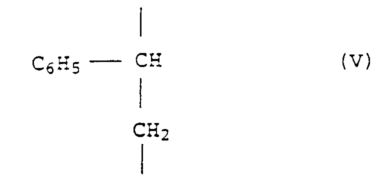 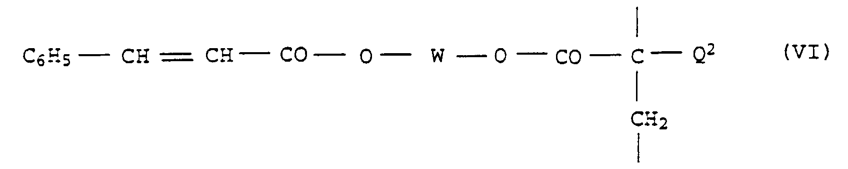 où
Q¹ représente un groupement hydroxy, alcoxy en C₁-C₆, oxirannylméthoxy, phénoxy, amino ou mono- ou di(alkyle en C₁-C₄)amino,
Q² représente un atome d'hydrogène ou un groupement méthyle et
W représente un groupement alkylène en C₂-C₁₀,
où la proportion des motifs monomères sous forme de reste bivalent dérivant de la formule I vaut 1-100% en moles, celle des restes de formule IV vaut 0-99% en moles, celle des restes de formule V vaut 0-99% en moles et celle des restes de formule VI vaut 0-75% en moles, à chaque fois par rapport au polymère, et la masse moléculaire moyenne du polymère vaut 1000-500000.

10. Utilisation de polymères contenant des colorants azoïques selon la revendication 9 en optique non-linéaire.
